# EUROPEAN PATENT SPECIFICATION

(11) **EP 3 368 906 B1**
(45) Date of publication and mention of the grant of the patent: **31.12.2025**
(21) Application number: 16801137.7
(22) Date of filing: 27.10.2016
(51) Int. Cl.: G01N 33/68

(54) **METHOD FOR PREDICTING CONTRAST INDUCED NEPHROPATHY**
VERFAHREN ZUR VORHERSAGE VON KONTRASTINDUZIERTER NEPHROPATHIE
MÉTHODE DE PRÉDICTION DE NÉPHROPATHIE INDUITE PAR CONTRASTE

(30) Priority: 29.10.2015 GB 201519142
(43) Date of publication of application: 05.09.2018
(73) Proprietor: Randox Laboratories Ltd., Crumlin, County Antrim BT29 4QY (GB)
(72) Inventor: MCENEANEY, David, Armagh BT63 5QQ (GB); LAMONT, John, Antrim BT29 4QY (GB)
(86) International application number: PCT/EP2016/075974
(87) International publication number: WO 2017/072252

(56) References cited:
- MALYSZKO JOLANTA ET AL: "Urinary and serum biomarkers after cardiac catheterization in diabetic patients with stable angina and without severe chronic kidney disease", RENAL FAILURE, INFORMA HEALTHCARE, US, vol. 31, no. 10, 1 January 2009 (2009-01-01), pages 910 - 919, XP009133887, ISSN: 1525-6049
- BACHORZEWSKA-GAJEWSKA H ET AL: "NGAL (neutrophil gelatinase-associated lipocalin) and L-FABP after percutaneous coronary interventions due to unstable angina in patients with normal serum creatinine", ADVANCES IN MEDICAL SCIENCES, UNIWERSYTET MEDYCZNY W BIALYMSTOKU, PL, vol. 54, no. 2, 1 December 2009 (2009-12-01), pages 221 - 224, XP002606190, ISSN: 1896-1126, [retrieved on 20091029], DOI: 10.2478/V10039-009-0036-1
- RUSSEL HIRSCH ET AL: "NGAL is an early predictive biomarker of contrast-induced nephropathy in children", PEDIATRIC NEPHROLOGY ; JOURNAL OF THE INTERNATIONAL PEDIATRIC NEPHROLOGY ASSOCIATION, SPRINGER, BERLIN, DE, vol. 22, no. 12, 14 September 2007 (2007-09-14), pages 2089 - 2095, XP019564418, ISSN: 1432-198X, DOI: 10.1007/S00467-007-0601-4
- D. BOLIGNANO ET AL: "Neutrophil Gelatinase-Associated Lipocalin (NGAL) and Progression of Chronic Kidney Disease", CLINICAL JOURNAL OF THE AMERICAN SOCIETY OF NEPHROLOGY, vol. 4, no. 2, 1 February 2009 (2009-02-01), pages 337 - 344, XP055006845, ISSN: 1555-9041, DOI: 10.2215/CJN.03530708

## Description

### Background

Contrast induced nephropathy (CIN) is an iatrogenic consequence of iodinated contrast media and accounts for a significant number of hospitalized Acute Kidney Injury (AKI) cases. AKI is a serious and common condition. A lack of consensus exists around the definition of AKI but typically it is defined as an abrupt loss of renal function that results in the retention of urea and other nitrogenous waste products often resulting in dysregulation of extracellular volume and electrolytes. Currently, creatinine is the most widely used biomarker for the detection of AKI. However, creatinine is a significantly delayed marker of renal injury and so is poor at early detection of acute tubular necrosis (ATN). Several classifications have been used to denote AKI as a result of CIN including the RIFLE (Risk, Injury, Failure, sustained Loss, End-stage renal disease) criteria. The incidence of AKI can depend on the causative agent, with AKI being reported in a variety of clinical settings including cardiopulmonary bypass surgery, sepsis, mechanical ventilation, kidney transplantation and the use of iodinated contrast dye. Patients undergoing PCI are amongst the highest risk group for development of CIN. Elderly patients, especially those with undiagnosed CKD (prevalence approximately 10-15% of this population) are at highest risk of cardiovascular morbidity and mortality. In one study, including over 7,000 consecutive patients, CIN (defined as ≥ 25% increase from baseline serum creatinine at 48hrs post PCI) developed in 13% patients with normal renal function and in 19% of patients with baseline CKD (GFR <60mls/min/1.73m²) (Dangas *et al.* 2005). A further study reported that CIN occurs in 14.5% of patients attending for coronary angiography. CIN prolongs hospital stay and affected patients demonstrate a 74% greater likelihood of being hospitalized for heart failure (McCullough *et al.* 1997).

The strongest risk factor for CIN development is the presence of pre-existent CKD. Chronic kidney disease (CKD) is a major public health concern. The incidence of CKD in the UK is 5-8% of the population. The prevalence of CKD increases exponentially with age, with the prevalence increasing further as a result in the significant rise in type two diabetes mellitus. The risk of mortality far outweighs the risk of end stage renal disease, as cardiovascular disease accounts for over 50% of deaths in this patient cohort, with CKD being an independent predictor of cardiovascular mortality. Other independent risk factors include increasing age (CIN incidence rises to 15% in patients >65 years), diabetic nephropathy, peripheral vascular disease, advanced cardiac failure and the use of non iso-osmolar contrast agents. Demographic changes towards a more aged population along with the increasing prevalence of diabetes mellitus (with commensurate increases in the prevalence of CKD and coronary artery disease) place detection of risk factors and prevention of CIN as a key health priority for both cardiac and renal services.

Mehran *et al.* (2004) published an important AKI risk score which has been adopted to identify those at highest risk of AKI-CIN. This scoring model assesses age, diabetes, anaemia, cardiac failure, dose of contrast, pre-procedure GFR, hypotension post procedure and use of intra-aortic balloon pump (IABP) to calculate risk score which subsequently equates to an individual patient's risk of CIN and dialysis. The risk of CIN exponentially rises over a score of ten, with one-year mortality also associated with a higher score.

The pathophysiology of CIN is yet to be fully determined but putative mechanisms have been proposed. One theory is intra-renal vasoconstriction of the afferent arteriole which decreases the glomerular capillary pressure, thus altering net filtration pressure and glomerular pressure. Contrast agents are thought to produce prolonged vasoconstriction of the afferent arteriole resulting in hypoxia, medullary ischaemic injury, and death of the proximal and distal renal tubular cells. There is no specific treatment for CIN once developed. Current interventions involve supportive care during the recovery phase and avoidance of further renal insults. The best treatment of CIN is prevention. Current therapeutic options are restricted to preventative strategies designed to mitigate CIN risk. Volume expansion has a well-established role in reducing the risk of CIN, although it is not clearly understood how volume expansion reduces the risk of CIN. In high risk patients (GFR <60mls/min) pre-hydration with either intravenous 0.9% saline or 1.4% sodium bicarbonate is indicated. A minimal dose of contrast agent should be administered as larger volumes of contrast have been associated with higher rates of CIN. In addition, the type of contrast used (depending on the iodine content) can affect rates of CIN.

Several biomarker studies have been undertaken in patient subgroups which appear promising as early predictors of CIN risk. Current biomarkers under investigation for early diagnosis of AKI include neutrophil gelatinase associated lipocalcin (NGAL), liver fatty acid-binding protein (L-FABP), kidney injury marker 1 (KIM-1), cystatin C (CysC), interleukin 18 (IL-18) and N-Acetyl-β-(D)-glucosaminidase (NAG).

NGAL is a 25kDa protease-resistant polypeptide that was initially identified bound to gelatinase in specific granules of the neutrophil. Plasma NGAL is highly accumulated in the human kidney cortical tubules, blood and urine following nephrotoxic and ischaemic injury. The value of NGAL in detecting AKI following iodinated contrast procedures such as diagnostic coronary angiography and/or PCI has not been fully studied. One study looked at 208 consecutive patients undergoing angiography or PCI with known diabetes and CKD. AKI was observed in 39 (18.8%) patients (Mori *et al.* 2005). A significant rise in serum NGAL was observed in the AKI positive group as early as 2 hours post contrast in the CIN arm (p=0.03) and this persisted at 4 hours (p=0.007) and 12-24 hours (p-0.0015). However, this group found no significant difference in urinary NGAL levels within the first 24 hours. Further studies in patients with normal renal function have also showed significantly higher levels of NGAL 2-4 hours following angiography in AKI compared with non-AKI patients (Bachorzewska-Gajewska *et al.* 2007; 2009 and Ling *et al.* 2008).

L-FABP is solely localized to the cytoplasmic region of the proximal tubular cells and selectively binds to intracellular free unsaturated fatty acids during hypoxic tissue injury. One study with patients undergoing PCI found L-FABP to be a relatively early marker of CIN, with urinary L-FABP levels significantly increased after 4 hours and remained raised until 48 hours post procedure (Bachorzewska-Gajewska *et al.* 2009). However, another study found that L-FABP was significantly higher in AKI patients only after 24 hours of contrast administration (p<0.01), with no statistically significant changes up to eight hours post procedure (Malyszko *et al.* 2009). A further study of stable CKD patients undergoing electively planned angiography found an AKI event rate of 8.6% (Manabe *et al.* 2012). Urinary L-FABP levels assessed before the angiogram and at one and two days after angiogram were found to be significantly raised pre-procedure in those patients who subsequently developed AKI by creatinine criteria, sensitivity 82%, specificity 69%, AUC 0.70, p=0.002. L-FABP levels at one and two days post procedure were also significantly higher in the AKI group, p=0.014 and p=0.003 respectively. Two further studies also found L-FABP significantly higher at 1-2 days in AKI patients (Nakamura *et al.* 2006; Kato *et al.* 2008).

With a move towards day case PCI, typically with discharge occurring six hours post procedure there is a need for an improved method to assess which patients are most at risk of developing CIN either before or soon after the procedure, thus facilitating early intervention to protect renal function, reduce morbidity, improve short and long-term prognosis and reduce length of hospital stay.

### References

Bachorzewska-Gajewska H, et al. Adv Med Sci (2009); 54:221-224.
Bachorzewska-Gajewska H, et al. Kidney Press Res (2007); 30:408-415.
Dangas G, et al. American Journal of Cardiology (2005); 95(1):13-9.
Kato K, et al. Circ J (2008); 72:1499-1505.
Ling W, et al. Nephron Clin Pract (2008); 108:176-181.
Malyszko J, et al. Renal Failure (2009); 31(10):910-9.
Manabe K, et al. European Journal of Clinical Investigation (2012); 42(5):557-63.
McCullough PA, et al. American Journal of Medicine (1997); 103(4):368-375.
Mehran R, et al. Journal of the American College of Cardiology (2004); 44(7):1393-9.
Nakamura T, et al. Am J Kidney Dis (2006); 47:439-444.
Qureshi AC, et al. Heart (2011); 97:A17-18.

### Figures

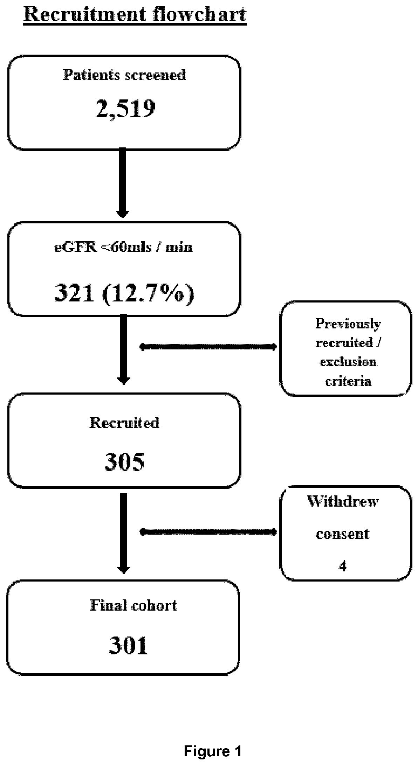

**Figure 1** Recruitment flowchart. A flowchart showing patients screened, excluded and the final cohort.
**Figure 2** Median values for biomarker levels at various time points before (0 hrs) and up to 48hrs after administration of contrast agent in AKI and Non-AKI patients, a) serum NGAL, b) urinary NGAL, c) serum L-FABP, d) serum KIM-1 and e) serum IL-18. Serum NGAL and serum L-FABP are significantly raised pre-contrast in AKI patients and remain elevated for several hours following contrast. Urinary NGAL only rises after 6 hours. KIM-1 and IL-18 were not predictive of AKI. An upper reference limit of 964ng/ml for NGAL and 12.1ng/ml for L-FABP were used in the analysis.
**Figure 3** % MACE / mortality events in AKI and non-AKI patients. MACE at one month, one year and mortality at one year are all significantly higher in AKI versus non-AKI patients

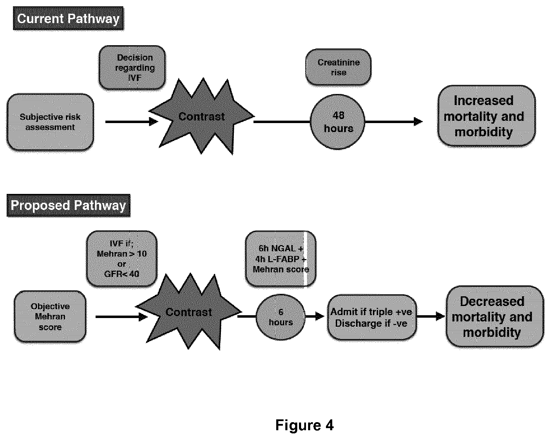

**Figure 4** Current and proposed patient pathways.

### Summary of the invention

The current invention provides a method for predicting the risk of developing contrast-induced nephropathy in patients with underlying chronic kidney disease according to claim 1. It is based on determining the concentrations of biomarkers in samples obtained from the patient and establishing the significance of these in relation to the likelihood of developing CIN. The biomarkers can be used in methods incorporating treatment protocols to minimise the risk of CIN developing once identified and also in methods to monitor progress of treatment and indicate risk of a major adverse clinical event occurring in future.

### Detailed description

In a first aspect the current disclosure provides a method for predicting the risk of developing contrast-induced nephropathy in a subject with underlying chronic kidney disease, said method comprising determining the concentrations of L-FABP and NGAL in an ex vivo serum sample obtained from the subject and establishing the significance of the biomarker concentrations.

The phrase 'underlying chronic kidney disease' refers to patients with pre-existing chronic kidney disease which may be diagnosed or undiagnosed and is the strongest risk factor for development of CIN. CKD as used herein refers to CKD as defined by the definition of CKD developed by the Kidney Disease Outcomes Quality Initiative (KDOQI):
1. Kidney damage present for at least 3 months, as defined by structural or functional abnormalities (most often based on increased albuminuria e.g. urinary albumin / creatinine ratio [UACR] ≥30 mg/g) and/or
2. Glomerular filtration rate (GFR) <60 mL/min/1.73 m2 present for at least 3 months.
   Within this framework, KDOQI then classified CKD into five stages, as follows:
   - Stage 1: Kidney damage with GFR ≥90 mL/min/1.73 m2.
   - Stage 2: Kidney damage with GFR 60-89 mL/min/1.73 m2.
   - Stage 3: GFR 30-59 mL/min/1.73 m2.
   - Stage 4: GFR 15-29 mL/min/1.73 m2.
   - Stage 5: GFR <15 mL/min/1.73 m2 or kidney failure treated by dialysis or transplantation.

In one preferred embodiment the sample is obtained from the subject ≤6 hours after administration of the contrast agent. In this embodiment the sample can be taken, for example, immediately after, 1hr after, 2hrs after, 3 hrs after, 4hrs after, 5hrs after or 6hrs after administration of contrast agent. In another preferred embodiment the sample is obtained from the subject ≤6 hours before administration of the contrast agent. In this embodiment the sample can be taken, for example, in hospital or clinic a few hours prior to undergoing a procedure requiring administration of contrast agent. In a further embodiment the sample can be taken immediately before administration of contrast agent. Multiple sample time points are also within the scope of the invention. For example, a sample could be taken at one time point then another at a later time point and levels of one or more biomarkers could be determined in each. These levels could be used to determine changes in biomarker levels over time or could be transformed into a ratio. Different biomarkers could be determined at different time points. For example, in one embodiment the level of L-FABP could be determined in a sample obtained from the patient 4 hours after administration of contrast media and the level of NGAL could be determined in a sample obtained from the patient 6 hours after administration of the contrast agent. However, in a medical facility it may be favourable to minimise the number of samples taken from the patient to limit the number of invasive procedures and reduce the workload for busy staff. Therefore, in a preferred embodiment both L-FABP and NGAL levels are measured in a sample obtained at a single time point.

As used herein, the term "sample" refers to a serum sample obtained from a patient or subject. The methods of the invention described herein are carried out *ex vivo.* For the avoidance of doubt, the term *"ex vivo"* has its usual meaning in the art, referring to methods that are carried out in or on a sample obtained from a subject in an artificial environment outside the body of the subject from whom the sample has been obtained. The terms "patient" and "subject" are used interchangeably herein and refer to any animal (e.g. mammal), including, but not limited to, humans, non-human primates, canines, felines, rodents and the like, which is to be the recipient of the diagnosis. Preferably, the subject or patient is a human.

The term "contrast agent" or "contrast medium" refers to any substance used to enhance the contrast of structures or fluids within the body during medical imaging. The contrast agent used in the methods of the current invention is preferably a non-ionic contrast agent such as Metrizamide, lohexol, loxaglic acid, lopamidol, lopromide, lotrolan, loversol, lopentol, lodixanol, lomeprol, lobitridol or loxilan. The methods of the invention are also applicable when an ionic contrast agent is used such as Diatrizoic acid, Metrizoic acid, lodamide, lotalamic acid, loxitalamic acid, loglicic acid, Acetrizoic acid, locarmic acid, Methiodal or Diodone. Other contrast agents include lodoxamic acid, lotroxic acid, loglycamic acid, Adipiodone, lobenzamic acid, lopanoic acid, locetamic acid, Sodium iopodate, Tyropanoic acid and Calcium iopodate. Most preferably the contrast agent is lodixanol.

The "level" of a biomarker refers to the amount, expression level or concentration of the biomarker within the sample. This level can be a relative level in comparison to another biomarker or a previous sample. The level of a biomarker may also refer to the biomarker measurement expressed as a ratio or percentage of the level of one or more other analytes. The level of one or more such other analytes may remain consistent in the majority of samples or conditions.

In a preferred embodiment of the current invention each of the biomarker concentration values is inputted into a statistical methodology to produce an output value that correlates with the chances that the patient has or is at risk of developing CIN. Preferably, the statistical methodology used is logistic regression, decision trees, support vector machines, neural networks, random forest or another machine-learning algorithm.

The performance of the results of the applied statistical methods used in accordance with the present invention can be described by their receiver operating characteristics (ROC). The ROC curve addresses both the sensitivity (the number of true positives) and the specificity (the number of true negatives) of the test. Therefore, sensitivity and specificity values for a given combination of biomarkers are an indication of the performance of the test. For example, if a biomarker combination has a sensitivity and specificity value of 80%, out of 100 patients, 80 will be correctly identified from the determination of the presence of the particular combination of biomarkers as positive for the disease, while out of 100 patients who do not have the disease 80 will accurately test negative for the disease.

One convenient goal to quantify the diagnostic accuracy of a laboratory test is to express its performance by a single number. The most common global measure is the area under the curve (AUC) of the ROC plot. The area under the ROC curve is a measure of the probability that the perceived measurement will allow correct identification of a condition. Values typically range between 1.0 (perfect separation of the test values of the two groups) and 0.5 (no apparent distributional difference between the two groups of test values), any result below 0.5 is considered to be worse than a random guess. The area does not depend only on a particular portion of the plot such as the point closest to the diagonal or the sensitivity at 90% specificity, but on the entire plot. This is a quantitative, descriptive expression of how close the ROC plot is to the perfect one (area = 1.0). In the context of the present invention, the two different conditions can be whether a patient has or does not have risk of developing CIN.

The ROC plot data and the clinical requirements of the test may be considered together when calculating a threshold or "cut-off" value to be used in future application of the diagnostic test. The cut-off value may also be referred to as the upper reference range and can be determined by consulting the manufacturer's instructions for a particular assay. When the analyte value is measured above (or below) this cut-off value, the test is considered "positive" and further action may be taken appropriate to the clinical condition. An important feature in setting the cut-off value is the required specificity of the test (i.e. the true positive rate). By convention, the required specificity for many diagnostic tests is stated in advance to be 90%, 95%, or as close to 100% as practical. The analyte cut-off value required to achieve these specificities may then be read from the ROC plot. This point on the plot will also denote a value for test sensitivity (true negative rate). Alternatively, the optimum cut-off value may be obtained by selecting the point on the ROC curve closest to the top-left corner of the graph. Another important feature is the negative predictive value (NPV) of the test, this is similar to the sensitivity as it describes the true negative rate but is also influenced by the disease prevalence in the population. A high NPV is important in conditions such as CIN as the clinician wants to be sure that a person being sent home following a procedure where a contrast agent has been administered has a low chance of developing CIN.

As described above, a cut-off value can be determined to give the required clinical performance for a diagnostic test such as high specificity or high NPV. For statistical analysis in the current invention cut-off values of 964 ng/ml for NGAL and 12.1 ng/ml for L-FABP were used. The skilled person will understand that altering these values can give equally informative results.

In the current invention levels of NGAL above a threshold value of about 900 ng/ml are indicative of a higher risk of developing CIN. In other preferred embodiment's levels of NGAL above about 1000 ng/ml, or above about 1100 ng/ml, or above about 1200ng/ml can also be used as cut-off values for risk of CIN giving high NPV. In the current invention levels of L-FABP above a threshold value of about 10 ng/ml are indicative of a higher risk of developing CIN. In further preferred embodiments L-FABP levels of above about 11 ng/ml or above about 12 ng/ml can also be used as cut-off values for a higher risk of developing CIN giving high NPV. The use of the word 'about' accounts for the fact that adjustments can be made to the cut-off values dependent on the required performance or assay used whilst still remaining within the grasp of the current invention.

A suitable statistical classification model, such as logistic regression, can be derived for a combination of biomarkers. Moreover, the logistic regression equation can be extended to include other (clinical) variables such as age and gender of the patient as well. In the same manner as described before, the ROC curve can be used to access the performance of the discrimination between patients and controls by the logistic regression model. Therefore, the logistic regression equation can be used apart or combined with other clinical characteristics to aid clinical decision making. Although a logistic regression equation is a common statistical procedure used in such cases and is preferred in the context of the current invention, other mathematical/statistical, decision trees or machine learning procedures can also be used.

In the context of the present invention, a "control" or "control value" is understood to mean the level of a biomarker typically found in patients who do not develop AKI. The control level of a biomarker may be determined by analysis of a sample isolated from a person who does not develop AKI or may be the level of the biomarker understood by the skilled person to be typical for such a person. The control value of a biomarker may be determined by methods known in the art and normal values for a biomarker may be referenced from the literature from the manufacturer of an assay used to determine the biomarker level.

The determination of the level of biomarkers in the sample may be determined by any method known to those skilled in the art, for example an immunological method such as an ELISA-based assay. The methods of the current invention preferably comprise the following steps; the biomarkers binding to a probe(s), adding a detector probe(s) and detecting and measuring the biomarker/probe complex signal(s), placing these values into a machine algorithm and analysing the output value, said value indicating whether the patient has or is at risk of developing CIN. Preferably, the methods of the present invention use a solid-state device for determining the level of biomarkers in the sample isolated from the patient.

The "solid-state device" comprises a substrate having a probe or multiple different probes immobilised upon it that bind specifically to a biomarker. The interactions between a biomarker and its respective probe can be monitored and quantified using various techniques that are well-known in the art. The term "probe" refers to a molecule that is capable of specifically binding to a target molecule such that the target molecule can be detected as a consequence of said specific binding. Probes that can be used in the present invention include, for example, antibodies, aptamers, phages and oligonucleotides. In a preferred embodiment of the current invention the probe is an antibody.

The term "antibody" refers to an immunoglobulin which specifically recognises an epitope on a target as determined by the binding characteristics of the immunoglobulin variable domains of the heavy and light chains (VHS and VLS), more specifically the complementarity-determining regions (CDRs). Many potential antibody forms are known in the art, which may include, but are not limited to, a plurality of intact monoclonal antibodies or polyclonal mixtures comprising intact monoclonal antibodies, antibody fragments (for example Fab, Fab', and Fv fragments, linear antibodies single chain antibodies and multispecific antibodies comprising antibody fragments), single-chain variable fragments (scFvs), multi-specific antibodies, chimeric antibodies, humanised antibodies and fusion proteins comprising the domains necessary for the recognition of a given epitope on a target. Preferably, references to antibodies in the context of the present invention refer to polyclonal or monoclonal antibodies. Antibodies may also be conjugated to various reporter moieties for a diagnostic effect, including but not limited to radionuclides, fluorophores, dyes or enzymes including, for example, horse-radish peroxidase and alkaline phosphatase.

Such antibodies may be immobilised at discrete areas of an activated surface of the substrate. The solid-state device may perform multi-analyte assays such that the level of a biomarker in a sample isolated from the patient may be determined simultaneously with the level of a further biomarker of interest in the sample. In this embodiment, the solid-state device has a multiplicity of discrete reaction sites each bearing a desired antibody covalently bound to the substrate, and in which the surface of the substrate between the reaction sites is inert with respect to the target biomarker. The solid-state, multi-analyte device may therefore exhibit little or no nonspecific binding. The combination of biomarkers may also be referred to as a panel of biomarkers.

The substrate can be any surface able to support one or more probes, but is preferably a biochip. A biochip is a planar substrate that may be, for example, mineral or polymer based, but is preferably ceramic. When identifying the various biomarkers/proteins of the invention it will be apparent to the skilled person that as well as identifying the full-length protein, the identification of a fragment or several fragments of a protein is possible, provided this allows accurate identification of the protein. Similarly, although a preferred probe of the invention is a polyclonal or monoclonal antibody, other probes such as aptamers, molecular imprinted polymers, phages, short chain antibody fragments and other antibody-based probes may be used.

A solid-state device that may be used in the invention may be prepared by activating the surface of a suitable substrate and applying an array of antibodies on to the discrete sites on the surface. If desired, the other active areas may be blocked. The ligands may be bound to the substrate via a linker. In particular, it is preferred that the activated surface is reacted successively with an organosilane, a bi-functional linker and the antibody. The solid-state device used in the methods of the present invention may be manufactured according to the method disclosed in, for example, GB-A-2324866. The solid-state device can be any substrate to which probes of the current invention can be attached for example a microtitre plate or beads. Preferably, the solid-state device used in the methods of the present invention is a biochip. The biochip may be a biochip which is incorporated into the Biochip Array Technology System (BAT) available from Randox Laboratories Limited (Crumlin, UK).

A preferred solid-state device would be a point-of-care device which can be placed in a medical facility and provide a medical professional with an output value indicating the risk of developing CIN for an individual patient.

The biomarker combination may be used in a not claimed method of reducing the likelihood of a patient with pre-existent CKD suffering CIN, said method comprising firstly determining the risk of developing CIN in said patient based on levels of one or more of L-FABP and NGAL and/or the Mehran risk score, then if the patient is determined to be at high risk of developing CIN administering a treatment or intervention to mitigate the risk. The treatment or intervention may be intravenous fluids or additional renal protective therapies including but not limited to, angiotensin-converting enzyme inhibitors or angiotensin receptor blockers. The intervention may also be or include a more prolonged observation period post procedure.

The biomarker combination may also be used to predict the risk of major adverse clinical events (MACE) in patients with CKD. The term MACE includes acute MI, heart failure, hospitalisation, stroke and death. Yet another potential use of the biomarker combination is the monitoring of treatment in patients suffering from CIN. A reduction in levels of either or both of L-FABP and NGAL would indicate that the treatment is effective.

### Methods

### Study design and patient selection

A prospective cohort study in a single cardiology centre in the United Kingdom was carried out (Craigavon Cardiac Centre, Northern Ireland). Regional ethical committee approval (11/NI/0153) in addition to research and development committee approval was granted. The trial was registered with an International Standardised Randomized Control Trial Number (ISRCTN 21423). Patients at high risk of CIN, evidenced by the presence of chronic kidney disease (baseline GFR by modified MDRD method ≤60mls/min) were identified prior to cardiac catheterisation. Patients with recent myocardial infarction, hospitalization or heart failure (within 6 weeks of recruitment) were excluded.

### Recruitment

2,519 patients were screened, of which 321 had GFR <60mls/min. Of these 321 patients 20 were excluded resulting in a final cohort of 301 patients. Patient demographics, risk factors, CKD stage, contrast used and dose of contrast administered were recorded. Written consent was obtained from all patients. A Mehran risk score with risk calculation of CIN and dialysis was calculated for each patient. Pre-procedural CIN prophylaxis with 0.9% saline was administered as per unit protocol. Non-ionic iso-osmolar contrast (lodixanol, trade name Visipaque) was used as standard for all patients.

### Laboratory analysis

Samples were collected via venepuncture directly prior to contrast angiogram and at 1, 2, 4, 6 and 48hr time points for time course analysis post catheterisation. Samples were centrifuged within 30 minutes of collection at 3,800 rpm for 5 minutes. Following centrifugation, the supernatant fluid was transferred into anonymously coded cryotubes and immediately frozen at -80 degrees Celsius. Directly prior to biochip analysis, samples were thawed in one freeze-thaw cycle at Randox Laboratories, Crumlin, Northern Ireland. Standard sandwich ELISA immunoassay techniques were applied. An 'Evidence Investigator' system with thermoshaker was used for analysis.

### Statistical analysis

Categorical variables are presented as total number (n) and percentage (%). Continuous variables are presented as mean and standard deviation (SD). Categorical variables were compared using Chi squared test. Continuous variables were compared using independent student t-test when parametric (normally distributed) and Mann Whitney U test when non-parametric (not normally distributed). Statistical significance was considered if p < 0.05. Data was analysed using SPSS. Logistic regression analysis was applied to AKI risk factors. An upper reference limit of 964ng/ml for NGAL (Randox laboratories Ltd.) and 12.1ng/ml for L-FABP (Pelsers Clinical chem) were used in the analysis.

In addition to the primary endpoint, patients were assessed for major adverse clinical events (MACE) at 30 days and 1 year which included acute MI, heart failure, hospitalisation, stroke and death.

### Results:

### AKI versus non-AKI comparison

Of the 301 recruited patients, 28 (9.3%) developed AKI as defined as a rise of >25% from baseline creatinine or a delta rise of ≥26.5µmol/l measured 48 hours post contrast. Twenty (71.4%) of the AKI patients were male compared with 150 (55.0%) of non-AKI patients, p = 0.012. Baseline demographics of AKI vs non-AKI patients are shown in table 1, p<0.005 was taken as statistically significant.

### Serum NGAL:

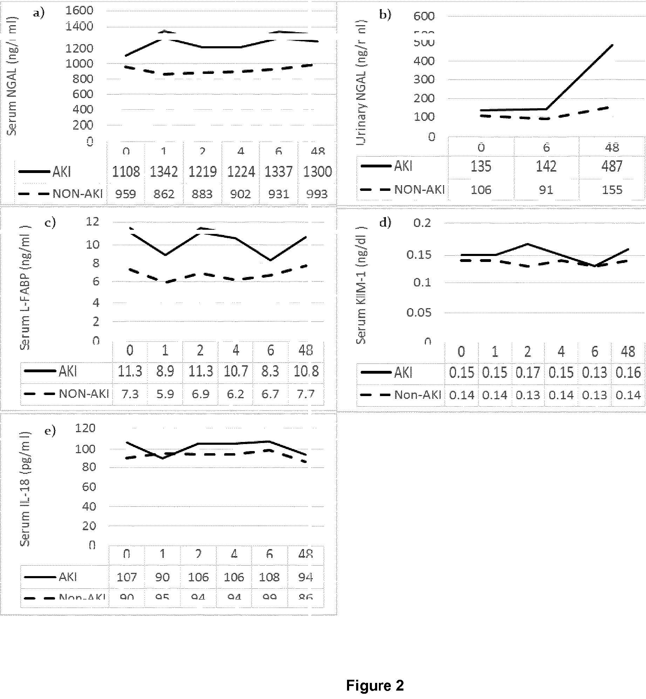

Median NGAL levels were significantly higher in AKI compared with non-AKI patients at all time-points, even prior to contrast administration. NGAL performed best at 6 hours post contrast with levels of 1,337ng/ml in AKI patients compared with 931ng/ml in non-AKI patients, p=0.002, AUC 0.71, sensitivity 75.0%, specificity 96.1%, PPV 11.5%, NPV 96.1%, RR 2.98, OR 2.86. A summary of comparisons between AKI and non-AKI patients at each time-point is shown in table 2 and figure 2.

### Serum L-FABP:

Similarly to serum NGAL, median L-FABP levels were significantly higher in AKI compared with non-AKI patients at all time-points, even prior to contrast administration. Serum L-FABP performed best at 4 hours with levels of 10.7ng/ml in AKI patients compared with 6.2ng/ml in non-AKI patients, p=0.001, AUC 0.69, sensitivity 42.3%, specificity 90.2%, PPV 31.4%, NPV 93.6%, RR 4.94, OR 6.75. A summary of comparisons between AKI and non-AKI patients at each time-point is shown in table 2 and figure 2.

### Urinary NGAL:

AKI patients had higher levels of urinary NGAL even at baseline. However, there was no significant rise in either group from baseline to 6 hours. Urinary NGAL levels dramatically increased between 6 and 48 hours in those who developed AKI compared with non-AKI patients, with median levels of 487ng/ml in AKI patients versus 155ng/ml in non-AKI patients, p=0.008, AUC 0.63, see figure 2.

### Urinary Cystatin C:

Median levels of urinary CysC were not different across both groups, despite p values <0.05. This is due to 0.4 being the lowest detectable value for the CysC assay used. Therefore, median levels at each time-point were <0.4 suggesting no signal across any time-point for AKI prediction.

### Serum KIM-1:

Median serum KIM-1 levels were not statistically different until 48 hours post contrast, with levels at this time-point of 0.16ng/ml in the AKI group versus 0.14ng/ml in the non-AKI group, p=0.019, AUC 0.65, see figure 2.

### Serum IL-18:

Serum IL-18 was not statistically significant at predicting AKI at any time-point, see figure 2.

### Mehran risk score

Median Mehran risk score was 11 in the AKI group and 8 in the non-AKI group, with a score ≥10 achieving an AUC of 0.65, p=0.006, sensitivity 64%, specificity 62%, PPV 10%, NPV 94%, RR 2.63 and OR 2.9 (Table 3).

Logistic regression analysis was performed showing diabetes, CKD stage and GFR were most associated with AKI.

### Exploratory analysis

From the results, serum NGAL, serum L-FABP and Mehran risk score were the best predictors of AKI. An assessment was made to determine if a combination of these biomarkers at various time-points and the clinical risk score could improve the diagnostic accuracy of AKI. A 4-hour L-FABP combined with Mehran score had the highest OR of 9.45 and highest RR of 5.99, AUC 0.65, p<0.001. A 6-hour NGAL combined with 4-hour L-FABP and Mehran score had the highest specificity at 96.4%, OR 6.27 (Table 4).

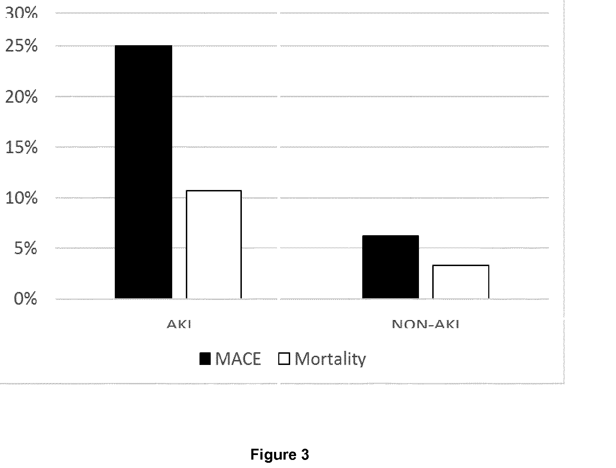

Major Adverse Clinical Event (MACE) / mortality:
At 1 year, MACE was observed in 7 (25%) AKI patients versus 17 (6.2%) non-AKI patients, p value <0.001. Mortality at one year was 3 (10.7%) in the AKI group compared with 8 (3.3%) in the non-AKI group, p = 0.037, see figure 3. Therefore, biomarkers could be used to predict adverse clinical outcomes in patients.

**Table 1: Baseline characteristics of AKI vs non-AKI patients**

| Demographic | AKI n=28 (9.3%) | Non-AKI n=273 (90.7%) | P value |
|---|---|---|---|
| | **n (%)** | | |
| Male | 20 (71.4) | 150 (55.0) | 0.102 |
| Hypertension | 28 (100) | 269 (98.5) | 0.648 |
| Hypercholesterolaemia | 22 (78.5) | 218 (79.6) | 0.843 |
| Smoker | 4 (14.2) | 28 (10.2) | 0.534 |
| DM | 13 (46.4) | 72 (26.4) | 0.026 |
| Stage 3A | 6 (21.4) | 162 (59.3) | <0.001 |
| Stage 3B | 5 (17.9) | 94 (34.4) | <0.001 |
| Stage 4 | 13 (46.4) | 13 (4.8) | <0.001 |
| Stage 5 | 4 (14.3) | 4 (1.5) | <0.001 |
| Heart Failure | 8 (28.6) | 59 (21.6) | 0.443 |
| Radial access | 25 (89.3) | 239 (87.5) | 0.683 |
| IVF administered | 16 (57.1) | 100 (36.6) | 0.211 |
| | | | |

| | **Mean (SD)** | | |
|---|---|---|---|
| Age (years) | 69.9 (10.1) | 73.9 (8.0) | 0.050 |
| Height (metres) | 1.7 (0.8) | 1.7 (0.2) | 0.254 |
| Weight (kilograms) | 81.7 (13.0) | 80.8 (18.3) | 0.643 |
| GFR (mls/min) | 29.8 (15.8) | 44.8 (10.0) | <0.001 |
| Creatinine (umol/l) | 212.9 (167.5) | 125.1 (39.0) | <0.001 |
| Mehran score | 10.8 (4.6) | 8.6 (4.0) | 0.008 |
| Contrast volume (mls) | 89.0 (51.1) | 68.1 (43.0) | 0.003 |
| Haemoglobin (g/dl) | 11.8 (1.6) | 13.0 (1.6) | <0.001 |
| White cell count (x10⁹/l) | 7.8 (2.3) | 7.4 (2.2) | 0.336 |

| | | | |
|---|---|---|---|
| *AKI: Acute Kidney Injury | | | |

**Table 2: Summary of median NGAL (ng/ml) and median L-FABP (ng/ml) levels in AKI and non-AKI groups**

| | Time (hr) | Median AKI | Median non-AKI | AUC | P value | Sensit (%) | Specif (%) | PPV (%) | NPV (%) | RR | OR |
|---|---|---|---|---|---|---|---|---|---|---|---|
| NGAL | 0 | 1108 | 959 | 0.62 | 0.046 | 56.0 | 49.6 | 9.6 | 92.2 | 1.22 | 1.25 |
| | 1 | 1342 | 862 | 0.68 | 0.003 | 64.0 | 56.8 | 13.3 | 94.3 | 2.35 | 2.56 |
| | 2 | 1219 | 883 | 0.68 | 0.004 | 64.0 | 54.7 | 12.1 | 94.0 | 2.00 | 2.14 |
| | 4 | 1224 | 902 | 0.65 | 0.014 | 57.7 | 56.4 | 12.4 | 92.6 | 1.66 | 1.76 |
| | 6 | 1337 | 931 | 0.71 | 0.002 | 75.0 | 96.1 | 11.5 | 96.1 | 2.98 | 2.86 |
| | 48 | 1300 | 993 | 0.69 | 0.003 | 70.8 | 94.2 | 11.1 | 94.2 | 1.92 | 2.03 |
| L-FABP | 0 | 11.3 | 7.3 | 0.65 | 0.011 | 44.0 | 87.4 | 21.2 | 94.0 | 3.48 | 4.14 |
| | 1 | 8.7 | 5.9 | 0.65 | 0.016 | 32.0 | 88.0 | 21.1 | 92.8 | 2.93 | 3.45 |
| | 2 | 11.3 | 6.9 | 0.67 | 0.007 | 50.0 | 86.0 | 25.5 | 94.7 | 4.82 | 6.14 |
| | 4 | 10.7 | 6.2 | 0.69 | 0.001 | 42.3 | 90.2 | 31.4 | 93.6 | 4.94 | 6.75 |
| | 6 | 8.3 | 6.7 | 0.63 | 0.045 | 33.3 | 86.9 | 18.4 | 93.6 | 2.89 | 3.32 |
| | 48 | 10.8 | 7.7 | 0.68 | 0.003 | 47.8 | 79.9 | 18.0 | 94.3 | 3.17 | 3.64 |

| | | | | | | | | | | | |
|---|---|---|---|---|---|---|---|---|---|---|---|
| Sensit: sensitivity; specif: specificity, PPV: positive predictive value, NPV: negative predictive value, RR: relative risk, OR: odds ratio | | | | | | | | | | | |

**Table 3: Mehran score performance for AKI prediction**

| | | | | | | | | |
|---|---|---|---|---|---|---|---|---|
| Mehran Risk score ≥10 | Sensit (%) | Specif (%) | PPV (%) | NPV (%) | P value | AUC | RR | OR |
| | 64 | 62 | 10 | 94 | 0.006 | 0.65 | 2.63 | 2.9 |

| | | | | | | | | |
|---|---|---|---|---|---|---|---|---|
| Sensit: sensitivity; specif: specificity, PPV: positive predictive value, NPV: negative predictive value, RR: relative risk, OR: odds ratio | | | | | | | | |

**Table 4: Exploratory analysis with combination testing**

| Combination test for AKI prediction | AUC | P value | Sensit (%) | Specif (%) | PPV (%) | NPV (%) | RR | OR |
|---|---|---|---|---|---|---|---|---|
| 0 hr NGAL + 0 hr L-FABP | 0.59 | 0.018 | 29.2 | 88.1 | 18.9 | 92.9 | 2.66 | 3.05 |
| 0 hr NGAL + 0 hr L-FABP + Mehran >10 | 0.60 | 0.001 | 25.0 | 94.0 | 28.6 | 92.9 | 4.05 | 5.29 |
| 2 hr NGAL + 2 hr L-FABP | 0.63 | <0.001 | 36.4 | 90.2 | 25.0 | 94.1 | 4.21 | 5.28 |
| 2 hr NGAL + 2 hr L-FABP + Mehran >10 | 0.60 | 0.001 | 37.5 | 93.8 | 28.6 | 93.8 | 6.07 | 5.67 |
| 4 hr NGAL + 4 hr L-FABP | 0.64 | <0.001 | 33.3 | 94.0 | 36.4 | 93.2 | 5.27 | 7.79 |
| 4 hr NGAL + 4 hr L-FABP + Mehran >10 | 0.61 | <0.001 | 40.0 | 95.7 | 40.0 | 92.5 | 5.33 | 8.22 |
| 6 hr NGAL + 6 hr L-FABP | 0.60 | 0.010 | 30.0 | 89.6 | 20.0 | 93.7 | 3.15 | 3.70 |
| 6 hr NGAL + 6 hr L-FABP + Mehran >10 | 0.57 | 0.021 | 20.0 | 93.9 | 22.2 | 93.1 | 3.23 | 3.87 |
| 6 hr NGAL + Mehran score >10 | 0.63 | 0.021 | 75.0 | 51.9 | 11.5 | 96.1 | 3.37 | 3.23 |
| 4 hr L-FABP + Mehran score >10 | 0.65 | <0.001 | 52.9 | 94.7 | 40.9 | 93.2 | 5.99 | 9.45 |
| 6 hr L-FABP + Mehran score >10 | 0.60 | 0.002 | 40.0 | 92.0 | 24.0 | 93.6 | 3.72 | 4.58 |
| 6 hr NGAL + 4 hr L-FABP + Mehran >10 | 0.57 | <0.002 | 19.0 | 96.4 | 33.0 | 88.8 | 4.50 | 6.27 |

| | | | | | | | | |
|---|---|---|---|---|---|---|---|---|
| Sensit: sensitivity; specif: specificity, PPV: positive predictive value, NPV: negative predictive value, RR: relative risk, OR: odds ratio, add biomarkers. | | | | | | | | |

## Claims

1. A method for predicting the risk of developing contrast-induced nephropathy (CIN) in a subject with pre-existent chronic kidney disease (CKD) undergoing a planned angiography or percutaneous coronary intervention, said method comprising:
a) Determining the concentration of liver fatty acid-binding protein (L-FABP) and neutrophil gelatinase-associated lipocalin (NGAL) in an *ex vivo* serum sample obtained from the subject
b) Establishing the significance of the biomarker concentrations by inputting each of the biomarker concentration values into a statistical methodology to produce an output value that correlates with the risk of developing CIN, wherein a level of NGAL ≥ about 900 ng/ml is indicative of an increased risk of developing CIN and a level of L-FABP ≥ about 10 ng/ml is indicative of an increased risk of developing CIN.

2. The method of claim 1 wherein L-FABP or NGAL are determined in an *ex vivo* serum sample obtained from the subject ≤ 6 hours after the subject had been administered contrast agent.

3. The method of claim 1 wherein L-FABP or NGAL are determined in an *ex vivo* serum sample obtained from the subject ≤ 6 hours before the subject had been administered contrast agent.

4. The method of claim 3 wherein L-FABP and NGAL are determined in an *ex vivo* serum sample obtained from the subject immediately before the subject had been administered contrast agent.

5. The method of claim 2 wherein NGAL is determined in a serum sample obtained from the subject 6hrs after the subject had been administered contrast agent and L-FABP is determined in a serum sample obtained from the subject 4hrs after the subject had been administered contrast agent.

6. The method of any previous claim wherein the additional steps of a') calculating the Mehran risk score for a subject and b') establishing the significance of the biomarker concentrations in combination with the Mehran risk score.

7. The method of claim 6 wherein a Mehran risk score ≥10 is indicative of an increased risk of developing CIN.

8. The method of any previous claim wherein a level of NGAL ≥ about 1200 ng/ml is indicative of an increased risk of developing CIN.

9. The method of any previous claim wherein a level of L-FABP ≥ about 12 ng/ml is indicative of an increased risk of developing CIN.

## Patentansprüche

1. Ein Verfahren zur Vorhersage des Risikos einer kontrastmittelinduzierten Nephropathie (CIN) bei Patienten mit vorbestehender chronischer Nierenerkrankung (CKD), die sich einer geplanten Angiographie oder perkutanen Koronarintervention unterziehen. Das Verfahren umfasst:
a) die Bestimmung der Konzentration von Leber-Fettsäure-bindendem Protein (L-FABP) und Neutrophilen-Gelatinase-assoziiertem Lipocalin (NGAL) in einer ex vivo Serumprobe des Patienten;
b) die Ermittlung der Signifikanz der Biomarker-Konzentrationen durch Eingabe der einzelnen Biomarker-Konzentrationswerte in ein statistisches Verfahren zur Erzeugung eines Ausgabewertes, der mit dem Risiko einer CIN korreliert. Ein NGAL-Wert ≥ ca. 900 ng/ml und ein L-FABP-Wert ≥ ca. 10 ng/ml deuten jeweils auf ein erhöhtes Risiko für eine CIN hin.

2. Verfahren nach Anspruch 1, wobei L-FABP oder NGAL in einer ex vivo Serumprobe bestimmt werden, die dem Probanden ≤ 6 Stunden nach der Verabreichung des Kontrastmittels entnommen wurde.

3. Verfahren nach Anspruch 1, wobei L-FABP oder NGAL in einer ex vivo Serumprobe bestimmt werden, die dem Probanden ≤ 6 Stunden vor der Verabreichung des Kontrastmittels entnommen wurde.

4. Verfahren nach Anspruch 3, wobei L-FABP und NGAL in einer ex vivo Serumprobe bestimmt werden, die dem Probanden unmittelbar vor der Verabreichung des Kontrastmittels entnommen wurde.

5. Verfahren nach Anspruch 2, wobei NGAL in einer Serumprobe bestimmt wird, die dem Probanden 6 Stunden nach der Verabreichung des Kontrastmittels entnommen wurde, und L-FABP in einer Serumprobe bestimmt wird, die dem Probanden 4 Stunden nach der Verabreichung des Kontrastmittels entnommen wurde.

6. Das Verfahren nach einem der vorhergehenden Ansprüche, wobei die zusätzlichen Schritte a') Berechnung des Mehran-Risiko-Scores für einen Probanden und b') Feststellung der Signifikanz der Biomarker-Konzentrationen in Kombination mit dem Mehran-Risiko-Score sind.

7. Verfahren nach Anspruch 6, wobei ein Mehran-Risiko-Score ≥ 10 auf ein erhöhtes Risiko für die Entwicklung einer CIN hinweist.

8. Verfahren nach einem der vorhergehenden Ansprüche, wobei ein NGAL-Spiegel ≥ ca. 1200 ng/ml auf ein erhöhtes Risiko für die Entwicklung einer CIN hinweist.

9. Verfahren nach einem der vorhergehenden Ansprüche, wobei ein L-FABP-Spiegel ≥ ca. 12 ng/ml auf ein erhöhtes Risiko für die Entwicklung einer CIN hinweist.

## Revendications

1. Une méthode de prédiction du risque de développer une néphropathie induite par un produit de contraste (NIC) chez un sujet atteint d'une maladie rénale chronique (MRC) préexistante et devant subir une angiographie ou une intervention coronarienne percutanée programmée. Cette méthode comprend :
a) la détermination de la concentration de la protéine de liaison aux acides gras hépatiques (L-FABP) et de la lipocaline associée à la gélatinase des neutrophiles (NGAL) dans un échantillon de sérum ex vivo prélevé chez le sujet ;
b) l'établissement de la signification statistique des concentrations de biomarqueurs par l'intégration de chaque valeur de concentration dans une méthode statistique afin de produire une valeur de sortie corrélée au risque de développer une NIC. Un taux de NGAL ≥ environ 900 ng/ml et un taux de L-FABP ≥ environ 10 ng/ml sont tous deux indicatifs d'un risque accru de développer une NIC.

2. Procédé selon la revendication 1, dans lequel le L-FABP ou le NGAL sont dosés dans un échantillon de sérum ex vivo prélevé chez le sujet ≤ 6 heures après l'administration du produit de contraste.

3. Procédé selon la revendication 1, dans lequel le L-FABP ou le NGAL sont dosés dans un échantillon de sérum ex vivo prélevé chez le sujet ≤ 6 heures avant l'administration du produit de contraste.

4. Procédé selon la revendication 3, dans lequel le L-FABP et le NGAL sont dosés dans un échantillon de sérum ex vivo prélevé chez le sujet immédiatement avant l'administration du produit de contraste.

5. Procédé selon la revendication 2, dans lequel le NGAL est dosé dans un échantillon de sérum prélevé chez le sujet 6 heures après l'administration du produit de contraste et le L-FABP est dosé dans un échantillon de sérum prélevé chez le sujet 4 heures après l'administration du produit de contraste.

6. Le procédé de toute revendication antérieure dans lequel les étapes supplémentaires a') calculent le score de risque Mehran pour un sujet et b') établissent la signification des concentrations de biomarqueurs en combinaison avec le score de risque Mehran.

7. Procédé selon la revendication 6, dans lequel un score de risque de Mehran ≥ 10 indique un risque accru de développer une néoplasie intraépithéliale cervicale (NIC).

8. Procédé selon toute revendication antérieure, dans lequel un taux de NGAL ≥ environ 1 200 ng/ml indique un risque accru de développer une NIC.

9. Procédé selon toute revendication antérieure, dans lequel un taux de L-FABP ≥ environ 12 ng/ml indique un risque accru de développer une NIC.
